# EUROPEAN PATENT SPECIFICATION

(11) **EP 4 684 402 B1**
(45) Date of publication and mention of the grant of the patent: **16.09.2026**
(21) Application number: 24710104.1
(22) Date of filing: 13.03.2024
(51) Int. Cl.: G16H 50/20, G16H 10/00, G16H 50/30

(54) **METHOD, DEVICE, SYSTEM AND COMPUTER PROGRAM FOR ASSESSING UNCERTAINTY OF A CLINICAL METRIC**
VERFAHREN, VORRICHTUNG, SYSTEM UND COMPUTERPROGRAMM ZUR BEURTEILUNG DER UNSICHERHEIT EINER KLINISCHEN METRIK
PROCÉDÉ, DISPOSITIF, SYSTÈME ET PROGRAMME INFORMATIQUE POUR ÉVALUER L'INCERTITUDE D'UNE MESURE CLINIQUE

(30) Priority: 22.03.2023 EP 23163551
(43) Date of publication of application: 28.01.2026
(73) Proprietor: Koninklijke Philips N.V., 5656 AE Eindhoven (NL)
(72) Inventor: PAPINI, Gabriele, 5656 AG Eindhoven (NL); VAN DER HEIDE, Esther Marjan, 5656 AG Eindhoven (NL); FERREIRA DOS SANTOS DA FONSECA, Pedro Miguel, 5656AG Eindhoven (NL)
(74) Representative: Philips Intellectual Property & Standards
(86) International application number: PCT/EP2024/056595
(87) International publication number: WO 2024/194100

(56) References cited:
- WO-A2-2018/162901
- CN-A- 115 486 824
- JOS VAN DER WESTHUIZEN ET AL: "Bayesian LSTMs in medicine", ARXIV.ORG, CORNELL UNIVERSITY LIBRARY, 201 OLIN LIBRARY CORNELL UNIVERSITY ITHACA, NY 14853, 5 June 2017 (2017-06-05), XP080767518
- THANVEER SHAIK ET AL: "Remote patient monitoring using artificial intelligence: Current state, applications, and challenges", ARXIV.ORG, CORNELL UNIVERSITY LIBRARY, 201 OLIN LIBRARY CORNELL UNIVERSITY ITHACA, NY 14853, 19 January 2023 (2023-01-19), XP091420245, DOI: 10.1002/WIDM.1485

## Description

### FIELD OF THE INVENTION

The present invention relates to a computer-implemented method, a device, a system and a computer program for assessing uncertainty of a clinical metric relating to a physiological state of a subject.

### BACKGROUND OF THE INVENTION

Monitoring cardiovascular and cardiorespiratory activities of a subject (e.g. a patient or other person) allows extracting clinical metrics (e.g. indexes for deterioration, nociception, and severity of a disorder) to assess the subject's state of health and to make clinical decisions based thereon. For instance, measuring the autonomic nervous system activity via heart rate variability features may be used to detect obstructive sleep apnea or to quantify the response to a nociceptive stimulus.

Clinical decisions are generally made based on an extracted metric as a single summary metric (e.g. the apnea-hypopnea index in the diagnosis of obstructive sleep apnea). Unfortunately, an erroneous extracted metric may lead to a wrong assessment of a subject's physiological state. Decisions regarding the treatment of the subject based on a wrongly assessed physiological state can be harmful or even fatal for a subject. The use of a single summary metric is therefore not necessarily sufficient for a user to decide on an appropriate treatment.

The extracted clinical metric is generally associated with a quality indication that indicates the reliability of the extracted clinical metric based on the physiological signals' characteristics (e.g. signal to noise ratio and missing values). However, the extracted clinical metric is also characterized by an uncertainty that is independent of the signal quality. The uncertainty independent of the signal quality can be subdivided into an aleatory uncertainty and an epistemic uncertainty.

Aleatory uncertainty represents the unknowns that vary randomly each time the same metric is extracted. Examples are measurement errors (e.g. random noise, electrical interference, poor electrode contact, etc.) that are not captured by the signal quality estimation, but also physiological behavior that is randomly or pseudo-randomly triggered (such as arrhythmias for metrics based on heart rate variability). The aleatory uncertainty thus represents the variability caused by randomness that cannot be controlled. An indication of aleatory uncertainty may be obtained by measuring the same phenomena several times in similar conditions and calculating the variability of the extracted metric (e.g. via standard deviation or interquartile range).

Epistemic uncertainty represents the lack of knowledge encoded in the method itself used to extract the (clinical) metric. The epistemic uncertainty represents the reliability of the extracted metric with respect to the knowledge limits of the used processing path (i.e. the series of processing steps that extract the clinical metric from the measured signals). For example, a processing path developed and tested for a certain patient population (having certain characteristics related to its state health) may have a high epistemic uncertainty when used for another patient population if the measured physiological signals and extracted metrics are different between the two patient populations.

The epistemic uncertainty may be estimated with repeated perturbations of processing steps for machine learning or deep learning methods. Gal et al. "Dropout as a bayesian approximation: Representing model uncertainty in deep learning", international conference on machine learning, PMLR, 2016, p. 1050-1059, discloses a theoretical framework casting dropout training in deep neural networks as approximate Bayesian inference in deep Gaussian processes. However, an epistemic uncertainty calculation may not be appropriate when the uncertainty does not lie in the machine learning part of the method (e.g. for feature-based machine learning approaches).

CN 115 486 824 A discloses a cuff-free blood pressure estimation system based on an uncertainty measurement method, which belongs to the field of signal processing and specifically comprises a signal acquisition module, a data preprocessing module, a feature extraction module and a blood pressure estimation module.

### SUMMARY OF THE INVENTION

It is an object of the present invention to provide a computer-implemented method, a device, a system and a computer program for an improved assessing of uncertainty of a clinical metric to improve patient safety by allowing for more sound clinical decisions.

The invention is defined by the claims.

In a first aspect of the present invention a method for assessing uncertainty of a clinical metric relating to a physiological state of a subject is presented, the method comprising:
obtaining one or more physiological signals relating to a physiological state of a subject;
processing the one or more physiological signals to determine a clinical metric by using two or more processing paths each comprising two or more processing steps, wherein the two or more processing paths differ from each other by at least one different processing step, each providing an intermediate output of the same type in the respective processing paths;
assessing the uncertainty of the determined clinical metric by comparing the intermediate outputs of the same type and determining the extent of difference between them; and
outputting the determined clinical metric and the assessed uncertainty of the determined clinical metric.

In a further aspect of the present invention a device for assessing uncertainty of a clinical metric relating to a physiological state of a subject is presented, the device comprising:
- a sensor input configured to obtain one or more physiological signals relating to the physiological state of the subject;
- a processor configured to:
   process the one or more physiological signals to determine a clinical metric by using two or more processing paths each comprising two or more processing steps, wherein the two or more processing paths differ from each other by at least one different processing step, each providing an intermediate output of the same type in the respective processing paths; and
   assess the uncertainty of the determined clinical metric by comparing the intermediate outputs of the same type and determining the extent of difference between them; and
- an information output configured to output the determined clinical metric and the assessed uncertainty of the determined clinical metric.

In another aspect of the present invention a system for assessing uncertainty of a clinical metric relating to a physiological state of a subject is presented, the system comprising:
- one or more sensors each configured to measure one or more physiological signals relating to the physiological state of the subject;
- a device as disclosed herein for assessing uncertainty of the clinical metric relating to the physiological state of a subject based on the measured one or more physiological signals; and
- a user interface configured to issue the estimated metric and the assessed uncertainty to a user.

In yet a further aspect of the present invention, there is provided a corresponding computer program comprising program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, computer program and medium have similar and/or identical preferred embodiments as the claimed method, in particular as defined in the dependent claims and as disclosed herein.

It has been found that methods to calculate clinical metrics from physiological (e.g. cardiovascular, cardiorespiratory) signals are always characterized by a degree of uncertainty. Part of this uncertainty, the so-called epistemic uncertainty, is related to the limited knowledge of the methods itself. The variability of a metric does not sufficiently reflect the reliability of the extracted (estimated) clinical metric. For instance, a low standard deviation of repeated estimates of the same metric does not necessarily imply that the average value of the metrics is reliable. However, to ensure appropriate treatment of a patient, users (e.g. hospital staff, a clinician) need to be sure whether they can "trust" a metric and make decisions based thereon. Therefore, there is a need to assess the epistemic uncertainty of an estimated metric to allow to make sound decisions on diagnosis and treatment of a subject.

The present invention is based on the idea that adding information about the epistemic uncertainty of a metric may help the user decide whether to use the metric as a basis to determine the treatment of a subject or not. Hence, the user may decide whether to determine the (further) treatment of the subject based solely on the metric or whether another parameter and/or another metric should be used as well to determine the treatment. If a metric (e.g. a nociception index) has a high uncertainty, the user may consider using another metric (e.g. another nociception index) or relying on a personal interpretation of recorded vital signs (e.g. heart rate and breathing rate). For example, if the estimation of the apnea-hypopnea index with a home sleep test has a low certainty, the user may consider an in-lab study with a complete polysomnographic recording.

Therefore, the present invention may allow a user to make more informed decisions on the treatment of patients and thus to increase patient safety. For this purpose, the present invention proposes to estimate the epistemic uncertainty associated with a clinical metric and presents ways how to do that.

The assessed uncertainty according to the present invention is independent of the estimated final value of the metric. Therefore, the assessed uncertainty may indicate the metric to be non-trustworthy although the metric extracted by different processing paths has zero or close to zero variability. The method according to the present invention differs substantially from methods generally used to assess an uncertainty or variability of a metric.

In contrast to "ensemble learning" methods calculating the variability of outputs from multiple machine learning or estimation systems trained on different data, the method according to the present invention is not data-driven and may therefore be used for a non-machine learning-based application as well. The method according to the present invention does not rely on training data as neural network systems do (to train a model). The uncertainty (and the clinical metric) may thus be assessed without prior "learning" and independent of the quality and/or amount of the training data.

Furthermore, the present invention may provide a direct estimation of the epistemic uncertainty. In contrast thereto, conventional methods providing an output probability (e.g. logistic regressions) which indicates the confidence of a model in performing an estimation do not quantify to what extent the model can be trusted in performing the estimation.

Also, methods with output metric rejection based on a variability obtained by calculating the metric from different methods provide an output variability related to aleatory uncertainty, not necessarily to epistemic uncertainty as it may be determined in the present invention.

Furthermore, methods with deep learning drop-out based epistemic uncertainty estimations, where processing steps within a deep learning model are randomly skipped and the variability of the output is estimated, are only applicable to neural network-like methods because in standard methods, processing steps cannot be "skipped". In contrast thereto, the method according to the present invention may be used in neural network-like applications. For example a neural network-like method may represent one of the processing steps of a processing path, or each layer (or group of layers) of the neural network-like method may represent a processing step (trained to have the same meaning). Furthermore, in contrast to so-called "end-to-end" neural network-like methods using raw input signals (or a preprocessed form of raw input signals) directly, the method according to the present may be used in hybrid methods comprising e.g. manually engineered features (different sets of features representing different processing paths) and/or neural network-like methods using the features as input. Thus, embodiments of the disclosed method may be used in non-neural network-like methods, in neural network-like methods and in hybrid methods.

While the invention is described in regard of clinical metrics, in particular for patient monitoring in a hospital setting or home care setting, it should be understood, that the herein described method, device, system and computer program may be used for a wide range of applications as well, i.e., may be used to assess the (epistemic) uncertainty of non-clinical metrics as well. Thus, the method according to the invention may be used in any device deriving a parameter based on algorithms where it is important to assess the performance and certainty of the parameter.

Also, it should be understood, that the method, device, system, and computer program may estimate more than one clinical metric and assess uncertainty of the estimated clinical metrics, respectively.

In the context of the present invention as disclosed herein, the term "metric" refers herein to a parameter which is extracted from one or more signals used to describe the status of a measured system (e.g. the patient). A clinical metric may be obtained for example from cardiovascular parameters, cardiorespiratory activity parameters, and/or physical activity parameters, etc., that describe specific characteristics of physiological signals such as electrocardiography (ECG) signals, photoplethysmography (PPG) signals, oronasal airflow signals or thoracoabdominal movements. For example, a nociception index obtained by processing of detected QRS complexes (combination of three of graphical deflections, the Q wave, the R wave and the S wave, representing ventricular depolarization) in an electrocardiogram signal may represent a clinical metric.

The term "processing step" refers herein to a step in which data or signals are processed and/or modified. Example for a processing step is extracting the QRS complexes from an electrocardiogram (ECG) signal with a specific detector or, in general, to calculate an intermediate output from an input signal with a specific method.

The term "processing path" refers herein to a set sequence of processing steps, i.e., a processing path comprises one or more processing steps in a set sequence. A processing path may be used to process a physiological signal in order to extract a clinical metric. In general, the processing paths used to extract the same clinical metric have the same sequence of processing step types. However, the processing paths may also comprise processing steps in a different order or merged processing steps.

To compare intermediate outputs of different processing paths, same processing steps may be treated as one merged processing step (in the respective processing path). For example, processing path P1 may comprise processing steps A1- B1-C1-D1-E1 (in this order) and processing path P2 may comprise processing steps A2-C2-B2-D2-E2 (in this order), wherein processing steps having the same letter provide an intermediate output of the same type. In this example, processing path P1 and processing path P2 comprise a switched sequence of processing steps B1 - C1 and C2 - B2, respectively. The intermediate outputs of the processing path P1 and processing path P2 may be compared to each other by comparing the intermediate output before and after the switched sequence of these processing steps. I.e., the intermediate outputs of the processing steps B1 and C2 may not be comparable to each other, but the intermediate output of the merged processing step B1 plus C1 may be compared to the intermediate output of the merged processing step C2 plus B2. Thus, the intermediate outputs of processing steps A1 and A2 may be compared to each other, and the intermediate outputs of processing steps C1 and B2 may be compared to each other (being both inputs to processing steps D1, D2, the intermediate outputs of the processing steps C1 and B2 may be of the same type). Further, in this example, the intermediate outputs of the non-switched processing steps D1 and D2 may be compared to each other, and the intermediate outputs of the non-switched processing steps E1 and E2 may be compared to each other.

The term "intermediate output" refers herein to the output of an (intermediate) processing step which is not the final output of the processing path, i.e., it is not the metric to be outputted (to a user).

The term "output of the same type" refers herein thereto, that the outputs may be compared to each other, i.e., in particular, have the same unit and the same "physiological meaning".

The method may additionally comprise the step of selecting processing steps composing the processing path. The specific processing path may be reused to calculate the metric (based on the same input and/or different input). The uncertainty of a clinical metric may thus be repeatably assessed by using the same processing path (having identical processing steps).

In one embodiment, assessing the uncertainty of the determined clinical metric comprises determining a total number of processing steps corresponding to the number of processing steps potentially providing different intermediate outputs and determining a number of processing steps providing substantially identical intermediate outputs and/or a number of processing steps not providing substantially identical intermediate outputs. Therefore, the uncertainty may take into account how many of the processing steps providing potentially different intermediate outputs of the same type do provide substantially identical intermediate outputs of the same type. Thus, the method may take into account a relative ratio of processing steps providing substantially identical intermediate outputs of the same type to the total number of processing steps. A relatively larger proportion of processing steps providing substantially identical intermediate outputs of the same type may indicate a lower uncertainty of the estimated metric. Therefore, the assessed uncertainty may indicate a more trustworthy metric when the number of substantially identical processing step outputs increases. The assessed uncertainty may decrease with an increasing number of processing paths (corresponding to more options to calculate the metric) only if the increase of different processing paths is accompanied by an increased number of substantially identical processing step outputs. Alternatively or additionally to determining the number of processing steps providing substantially identical intermediate output, the number of processing steps not providing substantially identical intermediate output may be determined for assessing the uncertainty of the determined clinical metric. The sum of the number of processing steps providing substantially identical intermediate output and the number of processing steps not providing substantially identical intermediate output equals the total number of processing step outputs. Therefore, the number of processing steps providing not substantially identical intermediate output may be used to take into account the relative ratio of processing steps providing substantially identical intermediate outputs of the same type, too.

In another embodiment, the method further comprises determining an uncertainty score for the determined clinical metric by determining a number of reductions corresponding to the number of processing steps providing substantially identical intermediate outputs in the corresponding processing paths, and calculating an uncertainty score using the total number of processing step outputs and the number of reductions.

Therefore, the number of processing steps may be reduced by treating intermediate outputs that are substantially identical as a single intermediate output. The uncertainty score for the determined clinical metric may be thus calculated using the total number of processing steps (i.e. with zero reductions) and the number of reductions. A user may assess the uncertainty and thus the reliability of the estimated metric by an (epistemic) uncertainty score. The uncertainty score may be for example calculated by dividing the difference between the total number of processing step outputs and the number of reductions by the sum of the total number of processing step outputs and the number of reductions. The uncertainty score may therefore take into account the relative proportion of processing steps providing substantially identical intermediate outputs. The uncertainty score may thus vary from zero to one, wherein zero indicates a low degree of uncertainty (corresponding to a trustworthy metric) and one indicates a high degree of uncertainty (corresponding to a not trustworthy metric). The determined uncertainty score may decrease (indicating a more trustworthy metric) if the number of reduction increases. With an increasing number of processing paths, the determined uncertainty score may only decrease if the increase of different processing paths is accompanied by an increased number of reductions.

In a further embodiment, the method further comprises identifying and indicating one or more processing steps which contribute the least in decreasing the uncertainty of the determined clinical metric. Therefore, the identified steps contributing the least in decreasing the uncertainty may be exchanged to increase the certainty of the determined metric. Thus, the extraction of the metric from a signal in regard of the processing path may be strategically improved to obtain an estimated metric with a low (epistemic) uncertainty. The user may opt for alternative measurements and metrics that are likely disjointed from the identified processing steps as well. If a processing step such as a heartbeat detection contributes the least in reducing the uncertainty, the input for the processing step such as the heart rhythm may be unreliable (e.g. due to arrhythmias being present causing a lowered beat detection performance) for calculating the clinical metric. The user may therefore consider other metrics (e.g. a respiration-based metric instead of a cardiovascular-based metric) and parameters and/or consider using an additional measurement. The additional measurement may be based on a more robust, albeit possible less comfortable, sensing modality (e.g. Holter ECG instead of a wearable photoplethysmography). Thus, the user may strategically select the basis (e.g. one or more metrics) on which the user makes the decisions on the treatment of the patient.

In one embodiment, the determined clinical metric and the assessed uncertainty is time dependent. Therefore, the clinical metric and the assessed uncertainty are functions of the time. Temporal progressions may thus be tracked and evaluated.

In another embodiment, at least two processing paths comprise at least one processing step of the same type. Therefore, two or more processing paths each comprise one or more processing steps which are equivalent to processing steps in the one or more other processing paths.

In a further embodiment, an intermediate output is different from another intermediate output if an absolute value of a difference between the intermediate outputs of the two intermediate outputs of the same type is equal or greater than a threshold value. The threshold may be predetermined, specified by the user and/or calculated according to a method. The threshold may be adapted during time (e.g. by the user or by recalculation) and/or may vary depending on specific metrics as well.

In one embodiment, an intermediate output is substantially identical to another intermediate output if an absolute value of a difference between the intermediate outputs of two intermediate outputs of the same type is smaller than a threshold value. The threshold may be predetermined, specified by the user and/or calculated according to a method. The threshold may be adapted during time (e.g. by the user or by recalculation) and/or may vary depending on specific metrics as well.

In a further embodiment, the method further comprises assessing and outputting the average and/or the standard deviation of the determined clinical metric using processing paths with one or more different processing steps. Therefore, the user may be provided with additional data to allow for more detailed assessment of the extracted metric. In particular, the user may assess to which degree the estimations for the clinical metric differ from each other, i.e., if the standard deviation of the estimations for the clinical metric is low, the two or more processing paths used to estimate the metric provide similar results. Preferably, the user may determine from the output whether the result of, e.g., one processing path differs strongly from the results of other processing paths used.

In one embodiment, the one or more physiological signals relate to cardiovascular parameters, cardiorespiratory parameters and/or physical activity parameters. The cardiovascular and/or cardiorespiratory parameters may be obtained in a clinical environment with patient monitoring or with home care.

In another embodiment, the one or more physiological signals comprise any of an electrocardiogram signal, a photoplethysmography signal, an oronasal airflow signal, a thoracoabdominal movements signal and/or a surrogate signal derived from the electrocardiogram signal, the photoplethysmography signal, the oronasal airflow signal and/or the thoracoabdominal movements signal.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention;
Fig. 2 shows a schematic diagram of an embodiment of a device according to the present invention;
Fig. 3 schematically shows an embodiment of a method according to the present invention;
Fig. 4 shows a flow chart of another embodiment of the method according to the present invention; and
Fig. 5A, 5B and 5C schematically show exemplary implementations of the method shown in Fig. 4.

### DETAILED DESCRIPTION OF EMBODIMENTS

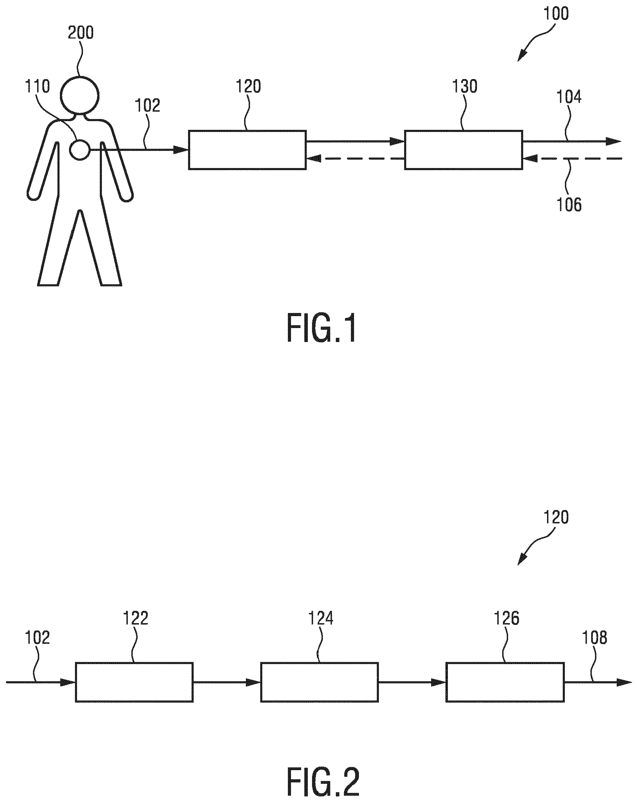

Fig. 1 schematically shows a measurement on a subject 200 (e.g. a patient in a hospital or a person in a nursing home or rest home) with a system 100 for assessing uncertainty of a clinical metric relating to a physiological state of the subject 200 according to an embodiment of the present invention. A sensor 110 (e.g. an ECG sensor, a PPG sensor, a movement sensor, etc.) is deployed on the subject 200 for acquiring or obtaining one or more physiological signals 102 (e.g. an ECG signal, a PPG signal, a movement signal, etc.) relating to the physiological state of the subject 200. The measured physiological signals 102 may then be processed by a device 120 (e.g. a processor or computer) to assess uncertainty of a clinical metric relating to the physiological state of the subject 200 based on the measured one or more physiological signals 102. The estimated metric and the assessed uncertainty may then be issued via a user interface 130 (e.g. a display of the device 120 or of a remote device, such as a central computer or workstation, or a patient monitor, to which the uncertainty and the physiological signals are provided) as a user output 104 to a user.

Optionally, the user interface 130 may obtain user input 106 comprising one or more user requests related to the estimation of the clinical metric and/or the assessment of the uncertainty of the estimated metric. The user interface 130 may then transmit the one or more user requests to the device 120 and the device 120 processes the one or more user requests accordingly.

Fig. 2 shows a schematic diagram of an embodiment of the device 120 for assessing the uncertainty of the clinical metric. The device 120 comprises a sensor input 122, a processor 124, and an information output 126. The sensor input 122 is configured to acquire or obtain the one or more physiological signals 102 from the sensor 110. The processor 124 may then process the obtained one or more physiological signals 102, determine (corresponding to an estimation of) the clinical metric based on the one or more physiological signals 102 and assess the uncertainty of the determined clinical metric. The determined (estimated) clinical metric and the assessed uncertainty may then be outputted by the information output 126 as an output 108.

The device 120 may e.g. be a patient monitor, a computer, a workstation, a tablet or smartphone, to which the physiological signals are provided, e.g. transmitted via a wired or wireless connection. The device 120 may have further processing capability to further process and output, e.g. display, the physiological signals.

Generally, an estimated clinical metric outputted to a user (e.g. a clinician, hospital staff, etc.) is characterized by an uncertainty. Usually, the outputted clinical metric is thus provided with an additional variability of the clinical metric (such as, e.g., the standard variation). The user often decides on the treatment of the subject based on such a single summary clinical metric. A low variability (e.g., a low stand variation) does, however, not necessarily imply a reliable average value of the clinical metric, i.e., the average value of the clinical metric is not necessarily accurate. To make sound clinical decisions, the user should know whether the outputted clinical metric is reliable or not. Wrongly estimated clinical metrics may lead to inappropriate decisions on treatment, being potentially harmful or even fatal for the subject.

The uncertainty of the clinical metric is generally partly dependent on the signal quality of the input (i.e., the physiological signal obtained from the subject) and partly independent from the signal quality of the input. The part independently from the signal quality may be further subdivided into aleatory uncertainty and epistemic uncertainty. The epistemic uncertainty represents the lack of knowledge encoded in a method itself to extract the clinical metric. The present invention presents ways how to assess the lack of knowledge encoded in the method itself, i.e., the epistemic uncertainty of the estimated clinical metric. One of the ideas of the present invention is to provide information on the epistemic uncertainty of an estimated clinical metric based on quantifying different processing paths used to process the same input physiological signal to determine (estimate) the same clinical metric.

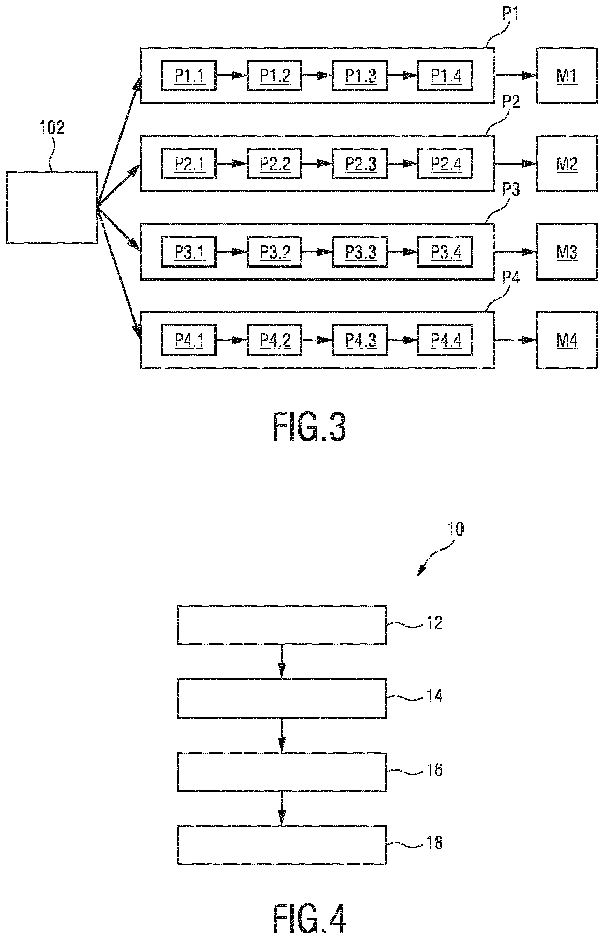

Fig. 3 shows a diagram schematically illustrating the extraction of a clinical metric from a physiological signal 102 (the input signal) according to an embodiment. The extraction may be performed by the device 120, and in particular by the input sensor 122 and the processor 124. The extraction may be implemented as a computer program running on a computer or processor. In the embodiment shown in Fig. 3, the clinical metric may be determined or estimated using four processing paths P1, P2, P3 and P4, each comprising four processing steps (P1.1 to P1.4, P2.1 to P2.4, P3.1 to P3.4 and P4.1 to P4.4). Using four processing paths may theoretically result in four different estimations for the clinical metric (e.g., a nociception index), but may also result in one, two or three different estimations. Less different estimations indicate a more reliable estimation as the estimation is more independent from the processing path used. The processing of the physiological input signal 102 using the (different) processing paths P1, P2, P3 and P4 results in the estimations M1, M2, M3 and M4 for the clinical metric. Depending on the used processing steps in the different processing paths P1, P2, P3 and P4, the value of the estimations M1, M2, M3 and M4 for the clinical metric may be all equivalent (i.e., M1=M2=M3=M4), similar (i.e., the values of M1, M2, M3 and M4 are almost the same) or different (i.e., the values of M1, M2, M3 and M4 differ significantly from each other).

The user may be presented with the one or more estimations for the clinical metric, accompanied by an average and/or standard deviation calculated from the different estimations using the four processing paths.

The four processing paths P1, P2, P3 and P4 shown in Fig. 3 are different in such a way that the processing paths differ from each other by at least one different processing step, i.e., each processing path differs from at least one other processing path by at least one processing step. The at least one different processing step provides an intermediate output of the same type in the respective processing path. Thus, the intermediate outputs (of the same type) may be compared to each other. The uncertainty of the estimated clinical metric may be then determined by comparing the intermediate outputs of the same type and determining the extent of difference between them (not shown in Fig. 3). In case of a low epistemic uncertainty, the result, i.e. the estimated clinical metric, should be independent of the processing path used.

Fig. 3 schematically shows processing steps for the estimation of one clinical metric based on one physiological signal 102 using four processing paths P1, P2, P3 and P4, each comprising four processing steps respectively. Generally, the input signal may comprise any number of physiological signals 102, i.e., one or more physiological signals 102. Also, any number of processing paths each with any number of processing steps may be used to extract the metric, i.e., two or more processing paths each comprising at least two processing steps. Furthermore, any number of different clinical metrics may be estimated in such a way, i.e., one or more clinical metrics.

Fig. 4 shows a flow chart of an embodiment of a method 10 for assessing the uncertainty of the clinical metric. The steps of the method 10 may be carried out by the device 120, wherein the main steps of the method 10 may be carried out by the processor 124. The method 10 may be implemented as a computer program running on a computer or processor (e.g. on the processor of a patient monitor).

In step 12, one or more physiological signals 102 relating to the physiological state of the subject 200 are obtained, e.g., retrieved or received, from the sensor 110. For instance, the physiological signals are provided via cable or wirelessly via Bluetooth or any other communication technology.

In step 14, the one or more physiological signals 102 are processed to determine a clinical metric by using two or more processing paths each comprising two or more processing steps, wherein the two or more processing paths differ from each other by at least one different processing step, i.e., each processing paths differs from at least one another processing path by at least one processing step, wherein the at least one different processing step provides an intermediate output of the same type in the respective (at least two) processing paths.

In step 16, the (epistemic) uncertainty of the determined clinical metric is determined by comparing the intermediate outputs of the same type (of the at least one different processing step) and determining the extent of difference between them. Finally, in step 18, the determined clinical metric and the assessed uncertainty of the determined clinical metric are outputted. The assessed uncertainty and the determined clinical metric may be outputted by the information output 126.

Optionally, the method 10 (e.g., step 16) may further comprise identifying and indicating one or more processing steps which contribute the least to decreasing the uncertainty of the determined clinical metric (not shown in Fig. 4), i.e., indicating a processing step which intermediate outputs differs significantly from processing path to processing path. Thus, one or more processing steps of a processing path may be exchanged (e.g., by using another detector) and/or a processing path discarded at all.

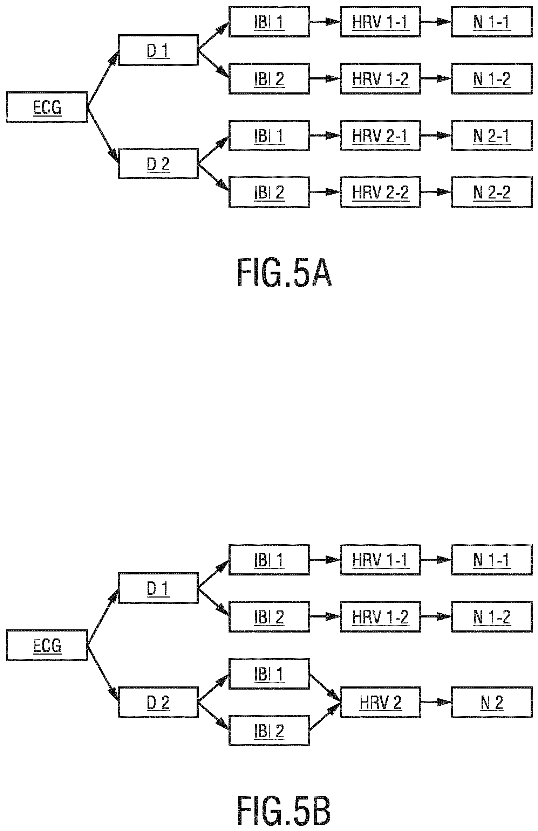

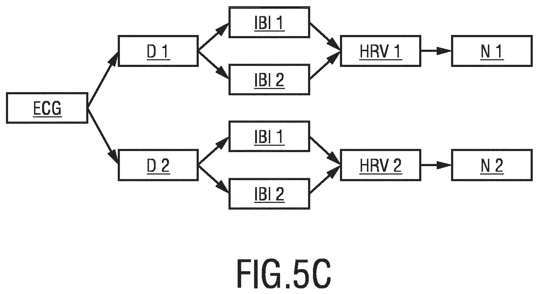

Fig. 5A, 5B and 5C show diagrams schematically illustrating the extraction of a nociception index (a clinical metric) from an ECG signal (a physiological signal 102) according to exemplary implementations of the method 10. The nociception index N may be obtained by extracting an ECG signal with two different QRS detectors, D1 and D2, and two different interbeat interval (IBI) calculations, IBI 1 and IBI 2, followed by a heart rate variability (HRV) feature extraction and a calculation step of the nociception index, resulting in an estimation of N.

Fig. 5A shows a processing tree comprising four processing paths, wherein each processing path gives a nociception index N1-1, N1-2, N2-1 and N2-2. Each processing path is composed of five processing steps: ECG acquisition, detector D1 or D2, interbeat interval calculation IBI1 or IBI2, HRV feature extraction and nociception determination. The sequence of the types of the processing step is equal for each processing path, i.e., for each processing path the ECG step is followed by the detector step, being followed by the IBI step, etc. Fig. 5A, 5B and 5C show three exemplary scenarios with different degrees of similarity between the intermediate step outputs of the five processing steps that compose each processing path. Due to the similarity, some of the processing paths may be "reduced" to one processing path such that less different nociception indices are obtained (some of the processing paths in Fig. 5B and 5C).

Fig. 5A shows a first exemplary scenario with four processing paths. There are no similar intermediate outputs of the same type. Processing with D1 and D2 and processing with IBI1 and IBI2 results in different values, respectively. The extraction of the ECG signal results in four different estimations of the nociception index, N1-1, N1-2, N2-1 and N2-2.

Fig. 5B shows a second exemplary scenario. For detector 2, the IBI obtained from the IBI 1 calculation and the IBI 2 calculation are similar. Intermediate outputs may be similar, e.g., if the difference between the (two values of) the intermediate outputs are lower than a certain threshold. The second exemplary scenario results in three different estimations of the nociception index, N1-1, N1-2 and N2.

Fig. 5C shows a third exemplary scenario. The IBI obtained from the IBI 1 calculation and IBI 2 calculation are similar for detector 1 and the IBI obtained from the IBI 1 calculation and the IBI 2 calculation are similar for detector 2. However, the extracted HRV features obtained via detector 1 and detector 2 are different. The third scenario thus results into two different estimations of the nociception index, N1 and N2.

Fig. 5A, 5B and 5C schematically show exemplary scenarios of an embodiment of the method 10 applied to the extraction of a nociception index from an ECG signal. However, the method 10 may be applied to other physiological signals 102 (or input signals in general) and clinical metrics (or metrics in general). Examples may be i) an apnea hypopnea index extracted from photoplethysmography, ii) a classification of sleep stages using EEG and/or cardiorespiratory information (e.g. ECG and additional respiratory movements), iii) an activity classification from one or more accelerometer signals, and iv) a deterioration prediction based on movements (e.g. measured by an accelerometer) and cardiovascular activity (e.g. heart rate).

The clinical metrics obtained in the scenarios shown in Fig. 5A, 5B and 5C are reliable to a different degree, i.e., the clinical metrics have a different uncertainty. The epistemic uncertainty of the clinical metrics may be assessed by determining the degree to which the processing steps providing intermediate output do provide similar output values independently of the actual processing step used. This allows to assess the uncertainty encoded in the method, i.e., in the processing steps, itself, corresponding to the epistemic uncertainty.

The method 10 shown in Fig. 4, e.g., the step 16, may further comprise determining a number of processing steps potentially providing different intermediate outputs and determining a number of processing steps providing substantially identical intermediate outputs (not shown in Fig. 4).

The method 10 may further comprise determining a number of reductions. Two or more processing paths may be determined to correspond to one reduced processing path by determining if the processing steps of two or more processing paths provide substantially identical intermediate outputs (of the same type) in the corresponding processing paths.

The method 10 may further comprise calculating an epistemic uncertainty score (EUS) of the clinical metric. The calculation may be carried out, e.g., in the step 16 of method 10 by the processor 124 of the device 120. The (epistemic) uncertainty of the clinical metric may be calculated by using the total number of processing step outputs and the number of reductions. The EUS may be calculated with, e.g., the following equation:$EUS = \frac{total number of processing step outputs - number of reductions}{total number of processing step outputs + number of reductions}$ wherein the EUS varies from zero to one, and wherein zero indicates a low degree of uncertainty (a "trustable" metric) and one indicates a high degree of uncertainty (a not "trustable" metric). However, the EUS may be calculated according to any mathematical equation that reflects the uncertainty of the estimated metric, in particular by taking into account the total number of processing step outputs and the number of reductions.

The difference between the scenarios shown in Fig. 5A, 5B and 5C may be quantified based on the total number of processing step outputs and the number of reductions due to the similarity between the intermediate processing step outputs. Each processing path shown in Fig. 5A, 5B and 5C comprises five processing steps. The total number of processing step outputs corresponds to the number of theoretically different processing step outputs of the processing tree. The processing tree may provide the following number of theoretically different processing step outputs:
(i) processing step ECG: 1
(ii) processing step detector: 2
(iii) processing step IBI: 4
(iv) processing step HRV feature: 4
(v) processing step calculation nociception index: 4

The sum of the theoretically different intermediate outputs is thus 1+2+4+4+4 = 15. Thus, the total number of processing step outputs for the scenarios shown in Fig. 5A, 5B and 5C is 15, respectively.

The number of reductions for the scenarios shown in Fig. 5A, 5B and 5C may be determined as follows:
(i) Fig. 5A: number of reductions = 5 + 0 (no similar intermediate outputs)
(ii) Fig. 5B: number of reductions = 5 + 1 +1 = 7 (HRV 2-1 and HRV 2-2 are reduced to HRV 2, and nociception indices 2-1 and 2-2 are reduced to nociception index 2)
(iii) Fig. 5C: number of reductions = 5 + 1 +1 + 1 +1 = 9 (HRV 1-1 and HRV 1-2 are reduced to HRV 1, HRV 2-1 and HRV 2-2 are reduced to HRV 2, nociception indices 1-1 and 1-2 are reduced to nociception index 2 and nociception indices 2-1 and 2-2 are reduced to nociception index 2).

Here, each of the five processing steps is considered to add the value one to the number of reductions respectively as there is always at least one output even if multiple processing steps have the same value. Therefore, the reduction for the scenario shown in Fig. 5A may be counted as number of reductions = 5, although none of the intermediate step outputs are similar.

The uncertainty scores amount therefore (i) $EUS = \frac{15 - 5}{15 + 5} = 0.5$ (Fig. 5A), (ii) $EUS = \frac{15 - 7}{15 + 7} = 0.36$ (Fig. 5B) and (iii) $EUS = \frac{15 - 9}{15 + 9} = 0.25$. Thus, the clinical metric determined in the third scenario shown in Fig. 5C provides the lowest uncertainty score, indicating a higher reliability than the estimated metric in the scenarios shown in Fig. 5A or 5B.

The method according to the present invention differs from methods generally used to determine uncertainty of the clinical metric. In particular, in contrast to methods generally used, the computational requirements for the method according to the present invention may be proportional to the obtained epistemic uncertainty. In the nociception example described above, the scenario shown in Fig. 5C (lowest uncertainty in this example) may need fewer resources (e.g. memory) than the scenario shown in Fig. 5A (highest uncertainty in this example).

Furthermore, in particular in contrast to methods with random components (e.g., sampling-based methods), the method according to the present invention is deterministic. Thus, repeated uncertainty estimation of the same (physiological) input signal will always result in the same epistemic uncertainty score.

Also, in contrast to generally used methods (e.g., end-to-end approaches), the method may provide the uncertainty contribution of each step of the clinical metric calculation.

In summary, the present invention provides a solution to assess of the epistemic uncertainty of a clinical metric. The assessed epistemic uncertainty of the clinical metric may allow for sound clinical decisions as the present invention may help a user to decide whether to base decisions on the estimated clinical metric, i.e., whether to "trust" the estimated clinical metric and to treat a patient accordingly. Thus, the present invention may help to improve patient safety.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (10) for assessing uncertainty of a clinical metric relating to a physiological state of a subject (200), the method (10) comprising:
obtaining one or more physiological signals (102) relating to a physiological state of a subject (200);
processing the one or more physiological signals (102) to determine a clinical metric by using two or more processing paths each comprising two or more processing steps, wherein the two or more processing paths differ from each other by at least one different processing step, each providing an intermediate output of the same type in the respective processing paths;
assessing the uncertainty of the determined clinical metric by comparing the intermediate outputs of the same type and determining the extent of difference between them; and
outputting the determined clinical metric and the assessed uncertainty of the determined clinical metric.

2. The computer-implemented method (10) as claimed in claim 1, wherein assessing the uncertainty of the determined clinical metric comprises:
determining a total number of processing step outputs corresponding to the number of processing steps potentially providing different intermediate outputs; and
determining a number of processing steps providing substantially identical intermediate outputs and/or a number of processing steps not providing substantially identical intermediate outputs.

3. The computer-implemented method (10) as claimed in claim 2, wherein assessing the uncertainty of the determined clinical metric comprises determining a relative ratio of processing steps providing substantially identical intermediate outputs of the same type to the total number of processing step outputs.

4. The computer-implemented method (10) as claimed in claim 2 or 3, further comprising determining an uncertainty score for the determined clinical metric by
determining a number of reductions corresponding to the number of processing steps providing substantially identical intermediate outputs in the corresponding processing paths; and
calculating an uncertainty score using the total number of processing step outputs and the number of reductions.

5. The computer-implemented method (10) as claimed in any one of the preceding claims, further comprising identifying and indicating one or more processing steps which contribute the least in decreasing the uncertainty of the determined clinical metric.

6. The computer-implemented method (10) as claimed in any one of the preceding claims, wherein the determined clinical metric and the assessed uncertainty are time dependent.

7. The computer-implemented method (10) as claimed in any one of the preceding claims, wherein at least two processing paths comprise at least one processing step of the same type.

8. The computer-implemented method (10) as claimed in any one of the preceding claims, wherein it is determined if an intermediate output is different from another intermediate output by determining if an absolute value of a difference between the intermediate outputs of the two intermediate outputs of the same type is equal to a threshold value or greater than a threshold value.

9. The computer-implemented method (10) as claimed in any one of the preceding claims, wherein it is determined if an intermediate output is substantially identical to another intermediate output by determining if an absolute value of a difference between the intermediate outputs of two intermediate outputs of the same type is smaller than a threshold value.

10. The computer-implemented method (10) as claimed in any one of the preceding claims, further comprising assessing and outputting the average and/or the standard deviation of the determined clinical metric using processing paths with one or more different processing steps.

11. The computer-implemented method (10) as claimed in any one of the preceding claims, wherein the one or more physiological signals (102) relate to cardiovascular parameters, cardiorespiratory parameters and/or physical activity parameters.

12. The computer-implemented method (10) as claimed in any one of the preceding claims, wherein the one or more physiological signals (102) comprise any of an electrocardiogram signal, a photoplethysmography signal, an oronasal airflow signal, a thoracoabdominal movements signal and/or a surrogate signal derived from the electrocardiogram signal, the photoplethysmography signal, the oronasal airflow signal and/or the thoracoabdominal movements signal.

13. Device (120) for assessing uncertainty of a clinical metric relating to a physiological state of a subject (200), the device (120) comprising:
- a sensor input (122) configured to obtain one or more physiological signals (102) relating to the physiological state of the subject (200);
- a processor (124) configured to:
process the one or more physiological signals (102) to determine a clinical metric by using two or more processing paths each comprising two or more processing steps, wherein the two or more processing paths differ from each other by at least one different processing step, each providing an intermediate output of the same type in the respective processing paths; and
assess the uncertainty of the determined clinical metric by comparing the intermediate outputs of the same type and determining the extent of difference between them; and
- an information output (126) configured to output the determined clinical metric and the assessed uncertainty of the determined clinical metric.

14. System (100) for assessing uncertainty of a clinical metric relating to a physiological state of a subject (200), the system (100) comprising:
- one or more sensors (110) each configured to measure one or more physiological signals (102) relating to the physiological state of the subject (200);
- a device (120) as claimed in claim 13 for assessing uncertainty of the clinical metric relating to the physiological state of the subject (200) based on the measured one or more physiological signals (102); and
- a user interface (130) configured to issue the estimated metric and the assessed uncertainty to a user.

15. Computer program comprising program code means for causing a computer to carry out the steps of the method (10) as claimed in any one of claims 1 to 12 when said computer program is carried out on the computer.

## Patentansprüche

1. Computerimplementiertes Verfahren (10) zum Beurteilen der Unsicherheit einer klinischen Metrik in Bezug auf einen physiologischen Zustand eines Subjekts (200), wobei das Verfahren (10) umfasst:
Gewinnen eines oder mehrerer physiologischer Signale (102) in Bezug auf einen physiologischen Zustand eines Subjekts (200);
Verarbeiten des einen oder der mehreren physiologischen Signale (102) zum Bestimmen einer klinischen Metrik unter Verwendung von zwei oder mehreren Verarbeitungspfaden, welche jeweils zwei oder mehr Verarbeitungsschritte umfassen, wobei sich die zwei oder mehreren Verarbeitungspfade durch zumindest einen verschiedenen Verarbeitungsschritt voneinander unterscheiden, wobei in den jeweiligen Verarbeitungspfaden jeder eine Zwischenausgabe vom selben Typ bereitstellt;
Beurteilen der Unsicherheit der bestimmten klinischen Metrik durch Vergleichen der Zwischenausgaben desselben Typs und Bestimmen des Ausmaßes einer Differenz zwischen ihnen; und
Ausgeben der bestimmten klinischen Metrik und Bestimmen der beurteilten Unsicherheit der bestimmten klinischen Metrik.

2. Computerimplementiertes Verfahren (10) nach Anspruch 1, wobei Beurteilen der Unsicherheit der bestimmten klinischen Metrik umfasst:
Bestimmen einer Gesamtzahl der Verarbeitungsschrittausgaben entsprechend der Zahl von Verarbeitungsschritten, welche potenziell verschiedene Zwischenausgaben bereitstellen; und
Bestimmen einer Zahl von Verarbeitungsschritten, welche im Wesentlichen identische Zwischenausgaben bereitstellen und/oder einer Zahl von Verarbeitungsschritten, welche keine im Wesentlichen identischen Zwischenausgaben bereitstellen.

3. Computerimplementiertes Verfahren (10) nach Anspruch 2, wobei Beurteilen der Unsicherheit der bestimmten klinischen Metrik Bestimmen eines relativen Verhältnisses von Verarbeitungsschritten, welche im Wesentlichen identische Zwischenausgaben desselben Typs bereitstellen, zur Gesamtzahl der Verarbeitungsschrittausgaben umfasst.

4. Computerimplementiertes Verfahren (10) nach Anspruch 2 oder 3, weiter umfassend Bestimmen eines Unsicherheitswerts für die bestimmte klinische Metrik durch:
Bestimmen einer Zahl von Reduktionen, welche der Zahl von Verarbeitungsschritten entspricht, welche im Wesentlichen identische Zwischenausgaben in den entsprechenden Verarbeitungspfaden bereitstellen; und
Berechnen eines Unsicherheitswerts unter Verwendung der Gesamtzahl von Verarbeitungsschrittausgaben und der Zahl von Reduktionen.

5. Computerimplementiertes Verfahren (10) nach einem der vorstehenden Ansprüche, ferner umfassend Identifizieren und Angeben eines oder mehrerer Verarbeitungsschritte, welche am wenigsten zum Verringern der Unsicherheit der bestimmten klinischen Metrik beitragen.

6. Computerimplementiertes Verfahren (10) nach einem der vorstehenden Ansprüche, wobei die bestimmte klinische Metrik und die beurteilte Unsicherheit zeitabhängig sind.

7. Computerimplementiertes Verfahren (10) nach einem der vorstehenden Ansprüche, wobei zumindest zwei Verarbeitungspfade zumindest einen Verarbeitungsschritt desselben Typs umfassen.

8. Computerimplementiertes Verfahren (10) nach einem der vorstehenden Ansprüche, wobei bestimmt wird, ob eine Zwischenausgabe von einer anderen Zwischenausgabe verschieden ist, indem bestimmt wird, ob ein absoluter Wert einer Differenz zwischen den Zwischenausgaben der zwei Zwischenausgaben desselben Typs gleich einem Schwellenwert oder größer als ein Schwellenwert ist.

9. Computerimplementiertes Verfahren (10) nach einem der vorstehenden Ansprüche, wobei bestimmt wird, ob eine Zwischenausgabe im Wesentlichen mit einer anderen Zwischenausgabe identisch ist, indem bestimmt wird, ob ein absoluter Wert einer Differenz zwischen den Zwischenausgaben von zwei Zwischenausgaben desselben Typs kleiner als ein Schwellenwert ist.

10. Computerimplementiertes Verfahren (10) nach einem der vorstehenden Ansprüche, ferner umfassend Beurteilen und Ausgeben des Mittelwerts und/oder der Standardabweichung der bestimmten klinischen Metrik unter Verwendung von Verarbeitungspfaden mit einem oder mehreren verschieden Verarbeitungsschritten.

11. Computerimplementiertes Verfahren (10) nach einem der vorstehenden Ansprüche, wobei sich das eine oder die mehreren physiologischen Signale (102) auf kardiovaskuläre Parameter, kardiorespiratorische Parameter und/oder Parameter der körperlichen Aktivität beziehen.

12. Computerimplementiertes Verfahren (10) nach einem der vorstehenden Ansprüche, wobei das eine oder die mehreren physiologischen Signale (102) eines von einem Elektrokardiogrammsignal, einem Photoplethysmographiesignal, einem oronasalen Luftstromsignal, einem Signal der thorakoabdominalen Bewegungen und/oder einem aus dem Elektrokardiogrammsignal, dem Photoplethysmographiesignal, dem oronasalen Luftstromsignal und/oder dem Signal der thorakoabdominalen Bewegungen abgeleiteten Surrogatsignal umfassen.

13. Vorrichtung (120) zum Beurteilen der Unsicherheit einer klinischen Metrik in Bezug auf einen physiologischen Zustand eines Subjekts (200), wobei die Vorrichtung (120) umfasst:
- einen Sensoreingang (122), welcher dazu konfiguriert ist, ein oder mehrere physiologische Signale (102) in Bezug auf den physiologischen Zustand des Subjekts (200) zu gewinnen;
- einen Prozessor (124), welcher dazu konfiguriert ist:
das eine oder die mehreren physiologischen Signale (102) zum Bestimmen einer klinischen Metrik unter Verwendung von zwei oder mehreren Verarbeitungspfaden zu verarbeiten, welche jeweils zwei oder mehrere Verarbeitungsschritte umfassen, wobei sich die zwei oder mehreren Verarbeitungspfade durch zumindest einen verschiedenen Verarbeitungsschritt voneinander unterscheiden, wobei in den jeweiligen Verarbeitungspfaden jeder eine Zwischenausgabe vom selben Typ bereitstellt; und
die Unsicherheit der bestimmten klinischen Metrik durch Vergleichen der Zwischenausgaben desselben Typs und Bestimmen des Ausmaßes einer Differenz zwischen ihnen zu beurteilen; und
- einen Informationsausgang (126), welcher dazu konfiguriert ist, die bestimmte klinische Metrik auszugeben und die beurteilte Unsicherheit der bestimmten klinischen Metrik zu bestimmen.

14. System (100) zum Beurteilen der Unsicherheit einer klinischen Metrik in Bezug auf einen physiologischen Zustand eines Subjekts (200), wobei das System (100) umfasst:
- einen oder mehrere Sensoren (110), welche dazu konfiguriert ist, ein oder mehrere physiologische Signale (102) in Bezug auf den physiologischen Zustand des Subjekts (200) zu messen;
- eine Vorrichtung (120) nach Anspruch 13 zum Beurteilen der Unsicherheit der klinischen Metrik in Bezug auf den physiologischen Zustand des Subjekts (200) basierend auf dem gemessenen einen oder mehreren physiologischen Signalen (102); und
- eine Benutzerschnittstelle (130), welche dazu konfiguriert ist, einem Benutzer die geschätzte Metrik und die beurteilte Unsicherheit herauszugeben.

15. Computerprogramm, umfassend Programmcodemittel zum Veranlassen eines Computers, die Schritte des Verfahrens (10) nach einem der Ansprüche 1 bis 12 auszuführen, wenn das Computerprogramm auf dem Computer ausgeführt wird.

## Revendications

1. Procédé mis en œuvre par ordinateur (10) pour l'évaluation de l'incertitude d'une mesure clinique relative à un état physiologique d'un sujet (200), le procédé (10) comprenant les étapes consistant à :
obtenir un ou plusieurs signaux physiologiques (102) relatifs à un état physiologique d'un sujet (200) ;
traiter les un ou plusieurs signaux physiologiques (102) pour déterminer une mesure clinique en utilisant deux trajets de traitement ou plus comprenant chacun deux étapes de traitement ou plus, dans lequel les deux trajets de traitement ou plus diffèrent les uns des autres par au moins une étape de traitement différente, chacune fournissant une sortie intermédiaire du même type dans les trajets de traitement respectifs ;
évaluer l'incertitude de la mesure clinique déterminée en comparant les sorties intermédiaires du même type et en déterminant l'ampleur de la différence entre elles ; et
délivrer en sortie la mesure clinique déterminée et l'incertitude évaluée de la mesure clinique déterminée.

2. Procédé mis en œuvre par ordinateur (10) selon la revendication 1, dans lequel l'évaluation de l'incertitude de la mesure clinique déterminée comprend les étapes consistant à :
déterminer un nombre total de sorties d'étape de traitement correspondant au nombre d'étapes de traitement fournissant potentiellement différentes sorties intermédiaires ; et
déterminer un certain nombre d'étapes de traitement fournissant des sorties intermédiaires sensiblement identiques et/ou un certain nombre d'étapes de traitement ne fournissant pas de sorties intermédiaires sensiblement identiques.

3. Procédé mis en œuvre par ordinateur (10) selon la revendication 2, dans laquelle l'évaluation de l'incertitude de la mesure clinique déterminée comprend une détermination d'un rapport relatif d'étapes de traitement fournissant des sorties intermédiaires sensiblement identiques du même type au nombre total de sorties d'étape de traitement.

4. Procédé mis en œuvre par ordinateur (10) selon la revendication 2 ou 3, comprenant en outre une détermination d'un score d'incertitude pour la mesure clinique déterminée en
déterminant un nombre de réductions correspondant au nombre d'étapes de traitement fournissant des sorties intermédiaires sensiblement identiques dans les trajets de traitement correspondants ; et
calculer un score d'incertitude en utilisant le nombre total de sorties d'étape de traitement et le nombre de réductions.

5. Procédé mis en œuvre par ordinateur (10) selon l'une quelconque des revendications précédentes, comprenant en outre une identification et une indication d'une ou plusieurs étapes de traitement qui contribuent le moins à diminuer l'incertitude de la mesure clinique déterminée.

6. Procédé mis en œuvre par ordinateur (10) selon l'une quelconque des revendications précédentes, dans lequel la mesure clinique déterminée et l'incertitude évaluée dépendent du temps.

7. Procédé mis en œuvre par ordinateur (10) selon l'une quelconque des revendications précédentes, dans lequel au moins deux trajets de traitement comprennent au moins une étape de traitement du même type.

8. Procédé mis en œuvre par ordinateur (10) selon l'une quelconque des revendications précédentes, dans lequel il est déterminé qu'une sortie intermédiaire est différente ou non d'une autre sortie intermédiaire en déterminant qu'une valeur absolue d'une différence entre les sorties intermédiaires des deux sorties intermédiaires du même type est égale à une valeur de seuil ou supérieure à une valeur de seuil.

9. Procédé mis en œuvre par ordinateur (10) selon l'une quelconque des revendications précédentes, dans lequel il est déterminé qu'une sortie intermédiaire est sensiblement identique ou non à une autre sortie intermédiaire en déterminant qu'une valeur absolue d'une différence entre les sorties intermédiaires de deux sorties intermédiaires du même type est inférieure à une valeur de seuil.

10. Procédé mis en œuvre par ordinateur (10) selon l'une quelconque des revendications précédentes, comprenant en outre une évaluation et une délivrance en sortie de la moyenne et/ou de l'écart type de la mesure clinique déterminée à l'aide de trajets de traitement comportant une ou plusieurs étapes de traitement différentes.

11. Procédé mis en œuvre par ordinateur (10) selon l'une quelconque des revendications précédentes, dans lequel les un ou plusieurs signaux physiologiques (102) se rapportent à des paramètres cardiovasculaires, des paramètres cardiorespiratoires et/ou des paramètres d'activité physique.

12. Procédé mis en œuvre par ordinateur (10) selon l'une quelconque des revendications précédentes, dans lequel les un ou plusieurs signaux physiologiques (102) comprennent un quelconque parmi un signal d'électrocardiogramme, un signal de photopléthysmographie, un signal de débit oronasal ou un signal de mouvements thoraco-abdominaux. et/ou un signal de substitution dérivé du signal d'électrocardiogramme, du signal de photopléthysmographie, du signal de débit oronasal et/ou du signal des mouvements thoraco-abdominaux.

13. Dispositif (120) pour l'évaluation de l'incertitude d'une mesure clinique relative à un état physiologique d'un sujet (200), le dispositif (120) comprenant :
- une entrée de capteur (122) configurée pour obtenir un ou plusieurs signaux physiologiques (102) relatifs à l'état physiologique du sujet (200) ;
- un processeur (124) configuré pour :
traiter les un ou plusieurs signaux physiologiques (102) pour déterminer une mesure clinique en utilisant deux trajets de traitement ou plus comprenant chacun deux étapes de traitement ou plus, dans lequel les deux trajets de traitement ou plus diffèrent les uns des autres par au moins une étape de traitement différente, chacune fournissant une sortie intermédiaire du même type dans les trajets de traitement respectifs ; et
évaluer l'incertitude de la mesure clinique déterminée en comparant les sorties intermédiaires du même type et en déterminant l'ampleur de la différence entre elles ; et
- une sortie d'information (126) configurée pour délivrer en sortie la mesure clinique déterminée et l'incertitude évaluée de la mesure clinique déterminée.

14. Système (100) pour l'évaluation de l'incertitude d'une mesure clinique relative à un état physiologique d'un sujet (200), le système (100) comprenant :
- un ou plusieurs capteurs (110) configurés chacun pour mesurer un ou plusieurs signaux physiologiques (102) relatifs à l'état physiologique du sujet (200) ;
- un dispositif (120) selon la revendication 13 pour l'évaluation de l'incertitude de la mesure clinique relative à l'état physiologique du sujet (200) sur la base des un ou plusieurs signaux physiologiques mesurés (102) ; et
- une interface utilisateur (130) configurée pour délivrer à un utilisateur la mesure estimée et l'incertitude évaluée.

15. Programme informatique comprenant des moyens de code de programme pour amener un ordinateur à mettre en œuvre les étapes du procédé (10) selon l'une quelconque des revendications 1 à 12, lorsque ledit programme informatique est mis en œuvre sur l'ordinateur.
